(19) Europäisches Patentamt
European Patent Office
Office européen des brevets

(11) Publication number: 0 400 858 B1

## (12) EUROPEAN PATENT SPECIFICATION

(45) Date of publication of patent specification: **02.11.95**   (51) Int. Cl.⁶: **A61K  7/30**

(21) Application number: **90305516.8**

(22) Date of filing: **22.05.90**

(54) **Cleansing commpositions.**

(30) Priority: **27.05.89 GB 8912270**

(43) Date of publication of application:
**05.12.90 Bulletin  90/49**

(45) Publication of the grant of the patent:
**02.11.95 Bulletin  95/44**

(84) Designated Contracting States:
**AT BE CH DE DK ES FR GB GR IT LI LU NL SE**

(56) References cited:
**EP-A- 0 248 936**
**EP-A- 0 253 772**
**EP-A- 0 323 049**

(73) Proprietor: **THE PROCTER & GAMBLE COM-
PANY**
**One Procter & Gamble Plaza**
**Cincinnati,**
**Ohio 45202 (US)**

(72) Inventor: **Young, Kenneth**
**71 Larksfield**
**Englefield Green,**
**Surrey TW20 0RA (GB)**

(74) Representative: **Brooks, Maxim Courtney et al**
**Procter & Gamble Limited**
**Whitley Road**
**Longbenton**
**Newcastle-upon-Tyne NE12 9TS (GB)**

**Description**

The present invention relates to cleansing compositions in granular-form, and especially to compositions for use in cleansing dentures and the like. In particular, the invention relates to granular-form compositions having more rapid and efficaceous denture cleansing performance and anti-bacterial activity.

Effervescent tablets and powders for cleansing dentures and the like are well known in the art. The aim of a denture cleanser product is to clean the denture as fully and as quickly as possible and especially to remove the accummulation of plaque, mucilageneous and bacterial deposits which collect while the denture is being worn. To wear a denture which has not been completely cleaned of plaque and bacterial deposits is not only unhygienic but can also within a short space of time result in a detrimental effect on the mucous membrane. Moreover bacterial deposits can lead to so-called bacterial corrosion of the plastics material used to produce the denture with consequent color-change and malodor-formation.

Accordingly, the present invention provides a powder or granular-form denture cleanser which cleanses more rapidly and which is more efficaceous on plaque, mucilageneous and bacterial deposits and has greater stain-removal performance and antimicrobial activity than those preparations which are presently known and available on the market. At the same time, the compositions provide improved aesthetics in terms of solution clarity and speckle/dye dissolution characteristics.

According to the invention, there is provided a granular denture cleansing composition comprising an inorganic persalt bleaching agent, an organic peroxyacid bleach precursor and an effervescence generator, wherein the composition comprises,

a) from 2% to 50% of a granular bleach activator composition having a pH in 1% aqueous dispersion in the range from 2 to 6.5 and comprising from 0.1% to 10% of an organic peroxyacid bleach precursor, and

b) from 30% to 98% of a granular bleach composition having a pH in the range from 7.5 to 10.5 and comprising from 5% to 70% of inorganic persalt bleaching agent.

All percentages and ratios herein are by weight of total composition, unless otherwise indicated.

The cleansing compositions of the invention thus comprise three essential components, a bleaching agent, a peroxyacid bleach precursor and an effervescence generator. Each of these will be discussed in turn.

The bleaching agent takes the form of an inorganic persalt and can be selected from any of the well-known bleaching agents known for use in denture cleansers such as the alkali metal and ammonium persulfates, perborates and perphosphates and the alkali metal and alkaline earth metal peroxides. Example of suitable bleaching agents include potassium, ammonium, sodium and lithium persulfates and perborate mono- and tetrahydrates, sodium pyrophosphate peroxyhydrate and magnesium, calcium, strontium and zinc peroxides. Of these, however, the alkali metal persulfates, perborates and mixtures thereof are highly preferred for use herein.

The amount of bleaching agent in the total composition is generally from 5 to 70% preferably from 10% to 50%. In the preferred compositions comprising the mixture of alkali metal persulfates and perborates, the overall persulfate:perborate ratio is preferably from 5:1 to 1:5, more preferably from 2:1 to 1:2.

The compositions herein also contain an organic peroxyacid precursor, which in general terms can be defined as a compound having a titre of at least 1.5 ml of 0.1 N sodium thiosulfate in the following peracid formulation test.

A test solution is prepared by dissolving the following materials in 1000 mls distilled water:sodium pyrophosphate

| $(Na_4P_2O_7.10H_2O)$ sodium perborate tetrahydrate | 2.5 g |
| $(NaBO_2.H_2O_2.3H_2O)$ having 10.4% available oxygen | 0.615 g |
| sodium dodecylbenzene sulphonate | 0.5 g |

To this solution at 60°C an amount of activator is added such that for each atom of available oxygent present one molecular equivalent of activator is introduced.

The mixture obtained by addition of the activator is vigorously stirred and maintained at 60°C. After 5 minutes from addition, a 100 ml portion of the solution is withdrawn and immediately pipetted onto a mixture of 250 g cracked ice and 15 ml glacial acetic acid. Potassium iodide (0.4 g) is then added and the liberated iodine is immediately titrated with 0.1 N sodium thiosulphate with starch as indicator until the first disappearance of the blue colour. The amount of sodium thiosulphate solution used in ml is the titre of the bleach activator.

The organic peracid precursors are typically compounds containing one or more acyl groups, which are susceptible to perhydrolysis. The preferred activators are those of the N-acyl or O-acyl compound type containing an acyl radical R-CO wherein R is a hydrocarbon or substituted hydrocarbon group having preferably from about 1 to about 20 carbon atoms. Examples of suitable peracid precursors include.

1) Acyl organoamides of the formula $RCONR_1R_2$, where RCO is carboxylic acyl radical, $R_1$ is an acyl radical and $R_2$ is an organic radical, as disclosed in US-A-3,117,148. Examples of compounds falling under this group include:

a) N,N - diacetylaniline and N-acetylphthalimide;

b) N-acylhydantoins, such as N,N'-diacetyl-5,5-dimethylhydantoin;

c) Polyacylated alkylene diamines, such as N,N,N'N'-tetraacetylethylenediamine (TAED) and the corresponding methylenediamine (TAMD) and hexamethylenediamine (TAHD) derivatives, as disclosed in GB-A-907,356, GB-A-907,357 and GB-A-907,358;

d) Acylated glycolurils, such as tetraacetylglycoluril, as disclosed in GB-A-1,246,338, GB-A-1,246,339 and GB-A- 1,247,429.

2) Acylated sulphonamides, such as N-methyl - N - benzoyl - menthane sulphonamide and N-phenyl-N-acetyl menthane sulphonamide, as disclosed in GB-A-3,183,266.

3) Carboxylic esters as disclosed in GB-A-836,988, GB-A-963,135 and GB-A-1,147,871. Examples of compounds of this type include phenyl acetate, sodium acetoxy benzene sulphonate, trichloroethylacetate, sorbitol hexaacetate, fructose pentaacetate, p - nitrobenzaldehyde diacetate, isopropeneyl acetate, acetyl aceto hydroxamic acid, and acetyl salicylic acid. Other examples are esters of a phenol or substituted phenol with an alpha-chlorinated lower aliphatic carboxylic acid, such as chloroacetylphenol and chloroacetylsalicylic acid, as disclosed in US-A-3,130,165.

4) Carboxylic esters having the general formula Ac L wherein Ac is the acyl moiety of an organic carboxylic acid comprising an optionally subsituted, linear or branched $C_6$-$C_{20}$ alkyl or alkenyl moiety or a $C_6$-$C_{20}$ alkyl-substituted aryl moiety and L is a leaving group, the conjugate acid of which has a pKa in the range from 4 to 13, for example oxybenzenesulfonate or oxybenzoate. Preferred compounds of this type are those wherein:

a) Ac is $R_3$-CO and $R_3$ is a linear or branched alkyl group containing from 6 to 20, preferably 6 to 12, more Preferably 7 to 9 carbon atoms and wherein the longest linear alkyl chain extending from and including the carbonyl carbon contains from 5 to 18, preferably 5 to 10 carbon atoms, $R_3$ optionally being substituted (preferably alpha to the carbonyl moiety) by Cl, Br, $OCH_3$ or $OC_2H_5$. Examples of this class of material include sodium 3,5,5,-trimethylhexanoyloxybenzene sulfonate, sodium 3,5,5-trimethylhexanoyloxybenzoate, sodium 2-ethylhexanoyl oxybenzenesulfonate, sodium nonanoyl oxybenzene sulfonate and sodium octanoyl oxybenzenesulfonate, the acyloxy group in each instance preferably being p-subsituted;

b) Ac has the formula $R_3(AO)_mXA$ wherein $R_3$ is a linear or branched alkyl or alkylaryl group containing from 6 to 20, preferably from 6 to 15 carbon atoms in the alkyl moiety, $R_5$ being optionally subsituted by Cl, Br, $OCH_3$ or $OC_2H_5$, AO is oxyethylene or oxypropylene, m is from 0 to 100, X is O, $NR_4$ or $CO-NR_4$, and A is CO, CO-CO, $R_6$-CO, $CO-R_6$-CO or $CO-NR_4$-$R_6$-CO wherein $R_4$ is $C_1$-$C_4$ alkyl and $R_6$ is alkylene, alkenylene, arylene or alkarylene containing from 1 to 8 carbon atoms in the alkylene or alkenylene moiety. Bleach activator compounds of this type include carbonic acid derivatives of the formula $R_3(AO)_mOCOL$,succinic acid derivatives of the formula $R_3OCO(CH_2)_2COL$, glycollic acid derivatives of the formula $R_3OCH_2COL$, hydroxypropionic acid derivatives of the formula $R_3OCH_2CH_2COL$, oxalic acid derivatives of the formula $R_3OCOCOL$, maleic and fumaric acid derivatives of the formula $R_3OCOCH=CHCOL$, acyl aminocaproic acid derivatives of the formula $R_3CONR_1(CH_2)_6COL$, acyl glycine derivatives of the formula $R_3CONR_1CH_2COL$, and amino-6-oxocaproic acid derivatives of the formula $R_3N(R_1)CO(CH_2)_4COL$. In the above, m is preferably from 0 to 10, and $R_3$ is preferably $C_6$-$C_{12}$, more preferably $C_6$-$C_{10}$ alkyl when m is zero and $C_9$-$C_{15}$ when m is non-zero. The leaving group L is as defined above.

5) Acyl-cyanurates, such as triacetyl- or tribenzoylcyanurates, as disclosed in US-A-3,332,882.

6) Optionally substituted anhydrides of benzoic or phthalic acid, for example benzoic anhydride, m-chlorobenzoic anydride and phthalic anhydride.

Of all the above, preferred are organic peracid precursors of types 1(c) and 4(a).

The level of peroxyacid bleach precursor by weight of the total composition is preferably from 0.1% to 10%, more preferably from 0.5% to 5%.

The effervescent generator of the compositions herein can be selected from generators which are effective under acid or alkaline pH conditions, but preferably it consists of a combination of a generator which is effective or most effective under acid pH conditions and a generator which is effective or most effective under alkaline pH conditions. Effervescence generators which are effective under acidic pH conditions include a combination of at least one alkali metal carbonate or bi-carbonate, such as sodium bicarbonate, sodium carbonate, sodium sesquicarbonate, potassium carbonate, potassium bicarbonate, or mixtures thereof, in admixture with at least one non-toxic, physiologically-acceptable organic acid, such as tartaric, fumaric, citric, malic, maleic, gluconic, succinic, salicylic, adipic or sulphamic acid, sodium fumarate, sodium or potassium acid phosphates, betaine hydrochloride or mixtures thereof. Effervescence generators which are effective under alkaline pH conditions include persalts such as alkali and alkaline earth metal perborates, persulphates, percarbonates, perphosphates and mixtures thereof as previously described but preferably it comprises a mixture of an alkali metal perborate (anhydrous, mono- or tetrahydrate) with a monopersulphate such as Caroat [R] marketed by E I du Pont de Nemours Co. and which is a 2:1:1 mixture of monopersulphate, potassium sulphate and potassium bisulphate and which has an active oxygen content of about 4.5%.

In highly preferred compositions, the bleach activator composition incorporates an effervescence generator which is effective or is most effective under acid pH conditions while the granular bleach composition incorporates an effervescence generator which is effective or is most effective under alkaline pH conditions. The combination of generators is important for achieving optimum dissolution characteristics and pH conditions for generation of the peroxyacid bleach and for achieving optimum cleaning and antimicrobial activity. The (bi)carbonate components generally comprise from 1% to 65%, preferably from 5% to 55% of the total composition; the acid components generally comprise from 1% to 50%, preferably from 5% to 40% of the total composition.

For optimum antiplaque and antibacterial performance, the cleansing composition is preferably designed in such a way that the alkaline bleach composition dissolves or disperses in water more slowly or later than the acidic bleach activator composition in order to provide, on placing the cleaning composition in water, an initial pH in the acidic range, preferably from 2 to 6.5 and especially from 3 to 5. Moreover, it is preferred that the alkaline composition be present in sufficient excess of the acidic composition in order to shift, upon completion of effervescence, the pH of the aqueous medium into the alkaline range, preferably to a pH of from 7.5 to 9, this pH being preferred for reasons of cleaning performance and solution clarity. The initial acidic pH should be maintained for a time from 5 seconds to 3 hours, preferably from 10 seconds to 60 minutes and especially from 15 seconds to 30 minutes. The relative proportions of alkaline and acidic compositions is generally in the range from 6:1 to 1:1, preferably from 4:1 to 2:1.

The slower dissolution rate of the alkaline composition compared with the acidic composition can be achieved in various ways, for example by the use of alkaline salts or compounds which are inherently sparingly or slowly soluble such as anhydrous sodium carbonate, calcium carbonate, calcium hydroxide, magnesium oxide, or magnesium hydroxide carbonate; or by converting at least a portion of the alkaline composition into a state of slower dissolution rate, for example by melting some of the alkaline composition components to form melt agglomerates followed by comminution to the desired particle size, or by compressing the alkaline composition components with a filler which has a slow dissolution rate such as anhydrous sodium sulfate and slowly water-soluble polymers, for example, proteins, cellulose ethers, cellulose esters, polyvinyl alcohol, alginic acid esters, vegetable fatty acid glycerides and other polymers having a colloidal or pseudo-colloidal character. Moreover, the relative rate of dissolution of the alkaline and acidic composition can be additionally controlled by appropriate formulation and distribution of the effervescent components of the solid base material. In particular, the acidic composition, at least, should incorporate both components of the carbonate or bicarbonate/acid effervescent couple, bicarbonate being highly preferred. The level of (bi)carbonate and acid components in the acid composition both generally lie in the range from 10% to 60% by weight thereof, and preferably from 15% to 50% thereof. In highly preferred compositions, the alkaline composition contains a proportion of bicarbonate (generally from 1% to 25%, preferably from 5% to 20% thereof) and acid (from 1% to 10% thereof) in addition to carbonate (from 10% to 60%, preferably from 25% to 45% thereof).

The bleach activator acid bleach composition components will generally also comprise a binder in a level of at least 5%, preferably from 10% to 50% by weight of the corresponding component granule. Suitable binders include polyvinylpyrrolidone, poly(oxyethylene) of molecular weight 20,000 to 500,000, polyethyleneglycols of molecular weight of from 1000 to 50,000, Carbowax having a molecular weight of from 4000 to 20,000, nonionic surfactants, fatty acids, sodium carboxymethyl cellulose, gelatin, fatty alcohols, phosphates and polyphosphate, clays, aluminosilicates and polymeric polycarboxylates. Of the above, polyethyleneglycols are highly preferred.

In a preferred process of preparing the denture cleansing compositions, the solid particulate bleach activator is mixed with the remaining ingredients of the bleach activator composition in a fluidizer, the mixture is raised to a temperature sufficient to liquify the binder (60-65°C in the case of the preferred polyethyleneglycol binders) and maintained under constant fluidization until granulation is complete. The mixture is then cooled under fluidization to below 30°C and seived through a 2 mm sieve. The alkaline bleach granule is prepared in identical manner. The sulphamic acid speckle system herein is prepared from sulphamic acid crystals of at least 600 $\mu$m (micron) sieve size by spraying the dye solution thereon in a penistaltic pump at an outlet air temperature of 40°-60°C. The acid and alkaline granules are then blended at the requisite ratio, and finally the flavor and speckle system are added.

The compositions of the invention can be supplemented by other usual components of cleansing tablet formulations, especially surfactants, chelating agents, enzymes, pigments/dyes, flavor oils such as oils of spearmint, peppermint and wintergreen, dyestuffs, sweeteners, foam depressants such as dimethylpolysiloxanes, foam stabilizers such as the fatty acid sugar esters, preservatives, lubricants such as talc, magnesium stearate, finely divided amorphous pyrogenic silicas, etc. The free moisture content of the final composition is desirably less than about 1% and especially less than about 0.5%.

The surface active agent used in the compositions of the invention can be selected from the many available that are compatible with the other ingredients of the denture cleanser, both in the dry state and in solution. Such materials are believed to improve the effectiveness of the other ingredients of the composition by aiding their penetration into the interdental surfaces. Also, these materials aid in the removal of food debris attached to the teeth. Between 0.1 and 5 percent by weight of the dry composition of a dry powder or granular anionic surface active agent, such as sodium lauryl sulfate, sodium N-lauroylsarcosinate, sodium lauryl sulfoacetate or dioctyl sodium sulfosuccinate or ricinoleyl sodium sulfosuccinate, may, for example, be included in the composition and preferably the surface active agent comprises between 2 and 4 percent of the composition.

Suitable cationic, non-ionic and ampholytic surface active agents include, for example, quaternary ammonium compounds such as cetyltrimethylammonium bromide, condensation products of alkylene oxides such as ethylene or propylene oxide with fatty alcohols, phenols, fatty amines or fatty acid alkanolamides, the fatty acid alkanol amides themselves, esters of long-chained ($C_8$-$C_{22}$) fatty acids with polyalcohols or sugars, for example glycerylmonostearate or saccharosemonolaurate or sorbitolpolyoxyethylenemono-or di-stearate, betaines, sulphobetaines or long-chain alkylaminocarboxylic acids.

Chelating agents beneficially aid cleaning and bleach stability by keeping metal ions, such as calcium, magnesium, and heavy metal cations in solution. Examples of suitable chelating agents include sodium tripolyphosphate, sodium acid pyrophosphate, tetrasodium pyrophosphate, aminopolycarboxylates such as nitrilotriacetic acid and ethylenediamine tetracetic acid and salts thereof, and polyphosphonates and aminopolyphosphonates such as hydroxyethanediphosphonic acid, ethylenediamine tetramethylenephosphonic acid, diethylenetriaminepentamethylenephosphonic acid and salts thereof. Highly preferred sequestrants herein include polymeric polycarboxylates such as homopolyacrylates comprising, on average, from 10 to 90, preferably from 20 to 80 acrylic monomer units, and maleic-acrylic copolymers having a maleic/acrylic mole ratio of from 1:4 to 4:1 and comprising on average from 100 to 400 acrylic monomer units. Polymeric polycarboxylates of this general type are found particularly valuable for providing solution clarity in the context of bleach activator denture cleansing compositions of the invention based on polyethyleneglycol as the binding agent. Advantageously, the chelating agent comprises between 0.1 and 60 percent by weight of the composition and preferably between 0.5 and 30 percent. Phosphonic acid chelating agents, however, preferably comprise, if present, from 0.1 to 1 percent, preferably from 0.1% to 0.5% by weight of composition. The denture cleansing compositions preferably comprise from 1% to 25% of a polymeric polycarboxylate sequestering agent.

The compositions of the invention may be coloured or included coloured speckles which may take the form of a dye or combination of dyes which are oxidizable and which act as time indicators becoming colourless or a different colour when cleaning is complete. Such dye stuffs include FDC blue 02, FDC red 03, FDC green 03 etc. In preferred embodiments, the composition comprises speckles in the form of a water-soluble or water-dispersible particulate carrier having bulk density of greater than 1.04 g/cc and which carries a surface coating of the dye or pigment. In highly preferred embodiments, the particulate carrier comprises crystals of sulphamic acid. Preferably, both the granular bleach activator composition and the granular bleach composition have a bulk density of less than 1.0 g/cc.

Enzymes suitable for use herein are exemplified by proteases, alkalases, amylases, lipases, dextranases, mutanases, glucanases etc.

The following Examples further describe and demonstrate the preferred embodiments within the scope of the present invention.

5

EXAMPLES I TO IV

The following are representative denture cleansing compositions according to the invention. The percentages are by weight of the total composition. The compositions are prepared as described earlier.

| | I | II | III | IV |
|---|---|---|---|---|
| **Acid Granule** | | | | |
| Tartaric Acid | – | – | 4 | – |
| Citric Acid | – | 6 | – | – |
| Sodium Carbonate | 1 | – | 2 | – |
| Sulphamic Acid | 9 | 4 | 6 | 5 |
| PEG 10,000 | 2.5 | – | 3 | 3 |
| PVP 40,000 | – | 3 | – | – |
| Sodium Bicarbonate | 8 | 9 | 7 | 8 |
| Sodium acid Pyrophosphate | – | – | – | 6 |
| Sodium tripolyphosphate | – | 3 | – | – |
| EDTA | 1.2 | 1.5 | 1 | 1 |
| TAED[1] | 2 | – | 3 | 1 |
| TMHOS[2] | – | 3 | – | 2 |
| EDTMP[3] | – | – | 1 | 1 |
| Sodium laurylsulphate | 0.5 | 1 | 0.6 | 0.8 |
| Polycarboxylate [5] | 1.2 | – | 3 | – |

6

**Alkaline Granule**

| Sodium Perborate | | | | |
|---|---|---|---|---|
| Monohydrate | 12 | 10 | 11 | 13 |
| Sodium Bicarbonate | 12 | 10 | 13 | 9 |
| Sulphamic acid | 4 | 3 | 3 | 2 |
| Sodium Tripolyphosphate | – | 5 | – | – |
| Sodium Pyrophosphate | – | – | 2 | – |
| PEG 20000 | 7 | 8 | 6 | – |
| PEO[4] | – | – | – | 7 |
| Caroat[R] | 10 | 9 | 12 | 8 |
| $C_{8-12}$ fatty acid glyceride | – | – | 2 | – |
| Sodium Carbonate | | to 100 | | |
| | | | | |
| Flavour | 1 | 1 | 1 | 1 |

**Colour Granule**

| Sulphamic acid | 8 | 6 | 7 | 8 |
|---|---|---|---|---|
| FDC Blue 02 | 0.02 | 0.02 | 0.02 | 0.02 |

1    Tetraacetylethylene diamine

2    Sodium 3,5,5-trimethylhexanoyloxybenzene sulfonate

3    Ethylenediaminetetramethylenephosphonic acid

4    Polyoxyethylene – molecular weight 100,000

5    Copolymer of 3:7 maleic/acrylic acid, average molecular weight about 70,000, as acid.

The denture cleansing compositions of Examples I to IV display more rapid and efficacious denture cleansing performance and bacterial activity than corresponding compositions which are free of peroxyacid bleach precursor.

**Claims**

1. A granular denture cleansing composition comprising an inorganic persalt bleaching agent, an organic peroxyacid bleach precursor and an effervescence generator, wherein the composition comprises (all percentages being by weight of total composition),

   a) from 2% to 50% of a granular bleach activator composition having a pH in 1% aqueous dispersion in the range from 2 to 6.5 and comprising from 0.1% to 10% of an organic peroxyacid bleach precursor, and

   b) from 30% to 98% of a granular bleach composition having a pH in the range from 7.5 to 10.5 and comprising from 5% to 70% of inorganic persalt bleaching agent.

2. A denture cleansing composition according to Claim 1 wherein the granular bleach activator composition comprises an effervescence generator effective under acid pH conditions which comprises both components of a carbonate or bicarbonate/acid effervescent couple such that the granular bleach composition dissolves or disperses in water more slowly than or later than the granular bleach activator composition in order to provide, on adding the denture cleansing composition to water, an initial pH in the acidic range.

3. A denture cleansing composition according to Claim 2 wherein the initial pH is from 2 to 6.5, preferably from 3 to 5 and is maintained within the acid range for from 5 seconds to about 3 hours, preferably from 10 seconds to 60 minutes, more preferably from 15 seconds to 30 minutes.

4. A denture cleansing composition according to any of Claims 1 to 3 wherein the granular bleach composition is present in sufficient excess of the bleach activator composition in order to shift, upon completion of effervescence, the pH of the aqueous medium into the alkaline range, preferably to a pH of from 7.5 to 9.

5. A denture cleansing composition according to any of Claims 1 to 4 wherein the granular bleach composition comprises an effervescence generator effective under alkaline pH conditions.

6. A denture cleansing composition according to any of Claims 1 to 5 wherein the inorganic persalt bleaching agent comprises one or more bleaching agents selected from alkali metal persulfates, alkali metal perborates and mixtures thereof.

7. A denture cleansing composition according to Claim 5 or 6 wherein the effervescence generator effective under alkaline pH conditions comprises an alkali metal perborate.

8. A denture cleansing composition according to any of Claims 1 to 7 wherein the organic peroxyacid bleach precursor is selected from acylated polyalkyldiamines, especially tetraacetylethylenediamine, and carboxylic esters having the general formula AcL wherein Ac is the acyl moiety of an organic carboxylic acid comprising an optionally substituted, linear or branched $C_6$-$C_{20}$ alkyl or alkenyl moiety or a $C_6$-$C_{20}$ alkyl-substituted aryl moiety and L is a leaving group, the conjugate acid of which has a pKa in the range from 4 to 13.

9. A denture cleansing composition according to any of Claims 1 to 8 wherein both the granular bleach activator composition and the granular bleach composition have a bulk density of less than 1.0 g/cc.

10. A denture cleansing composition according to any of Claims 1 to 9 comprising from 1% to 25% of a polymeric polycarboxylate sequestering agent.

11. A denture cleansing composition according to any of Claims 1 to 10 comprising speckles in the form of a water-dispersible particulate carrier having a bulk density of greater than 1.04 g/cc and carrying a surface coating of a dye or pigment.

12. A denture cleansing composition according to Claim 11 wherein the particulate carrier comprises crystals of sulphamic acid.

**Patentansprüche**

1. Körnige Zusammensetzung zur Reinigung von künstlichen Gebissen, umfassend ein anorganisches Persalzbleichmittel, einen organischen Peroxysäurebleichmittelprecursor und ein das Aufbrausen hervorrufendes Mittel, worin die Zusammensetzung
   a) 2% bis 50% einer körnigen Bleichmittelaktivatorzusammensetzung, welche einen pH-Wert in 1%iger wäßriger Dispersion im Bereich von 2 bis 6,5 aufweist und 0,1% bis 10% eines organischen Peroxysäurebleichmittelprecursors umfaßt, und
   b) 30% bis 98% einer körnigen Bleichmittelzusammensetzung, welche einen pH-Wert im Bereich von 7,5 bis 10,5 aufweist und 5% bis 70% eines anorganischen Persalzes als Bleichmittel enthalt,
   umfaßt (wobei sich alle Prozentsätze auf das Gewicht der Gesamtzusammensetzung beziehen).

**2.** Zusammensetzung zur Reinigung von künstlichen Gebissen nach Anspruch 1, worin die körnige Bleichmittelaktivatorzusammensetzung ein das Aufbrausen hervorrufendes Mittel umfaßt, welches unter sauren pH-Wert-Bedingungen wirksam ist, das beide Komponenten eines das Aufbrausen hervorrufenden Carbonat oder Bicarbonat/Säure-Paares enthält, so daß sich die körnige Bleichmittelzusammensetzung in Wasser langsamer oder später als die körnige Bleichmittelaktivatorzusammensetzung löst oder darin dispergiert wird, um bei Zugabe der Zusammensetzung zur Reinigung von künstlichen Gebissen in Wasser einen Anfangs-pH-Wert im sauren Bereich zu gewährleisten.

**3.** Zusammensetzung zur Reinigung von künstlichen Gebissen nach Anspruch 2, worin der Anfangs-pH-Wert von 2 bis 6,5, vorzugsweise von 3 bis 5 beträgt und innerhalb des sauren Bereiches während 5 Sekunden bis etwa 3 Stunden, vorzugsweise von 10 Sekunden bis 60 Minuten, stärker bevorzugt von 15 Sekunden bis 30 Minuten gehalten wird.

**4.** Zusammensetzung zur Reinigung von künstlichen Gebissen nach einem der Ansprüche 1 bis 3, worin die körnige Bleichmittelzusammensetzung gegenüber der Bleichmittelaktivatorzusammensetzung in ausreichendem Überschuß vorliegt, um nach Beendigung des Aufbrausens den pH-Wert des wäßrigen Mediums in den alkalischen Bereich, vorzugsweise zu einem pH-Wert von 7,5 bis 9 zu verschieben.

**5.** Zusammensetzung zur Reinigung von künstlichen Gebissen nach einem der Ansprüche 1 bis 4, worin die körnige Bleichmittelzusammensetzung ein das Aufbrausen hervorrufendes Mittel umfaßt, welches unter alkalischen pH-Wert-Bedingungen wirksam ist.

**6.** Zusammensetzung zur Reinigung von künstlichen Gebissen nach einem der Ansprüche 1 bis 5, worin das anorganische Persalzbleichmittel ein oder mehrere Bleichmittel umfaßt, welche unter Alkalimetallpersulfaten, Alkalimetallperboraten und Gemischen hievon ausgewählt sind.

**7.** Zusammensetzung zur Reinigung von künstlichen Gebissen nach Anspruch 5 oder 6, worin das das Aufbrausen hervorrufende Mittel, welches unter alkalischen pH-Wert-Bedingungen wirksam ist, ein Alkalimetallperborat umfaßt.

**8.** Zusammensetzung zur Reinigung von künstlichen Gebissen nach einem der Ansprüche 1 bis 7, worin der organische Peroxysäurebleichmittelprecursor unter acylierten Polyalkyldiaminen, insbesondere Tetraacetylethylendiamin, und Carbonsäureestern der allgemeinen Formel AcL ausgewählt ist, worin Ac den Acylrest einer organischen Carbonsäure darstellt, welche einen wahlweise substituierten, linearen oder verzweigten $C_6$-$C_{20}$-Alkyl- oder -Alkenylrest oder einen $C_6$-$C_{20}$-Alkyl-substituierten Arylrest umfaßt und L eine Leaving-Gruppe darstellt, deren konjugierte Säure einen pKa-Wert im Bereich von 4 bis 13 aufweist.

**9.** Zusammensetzung zur Reinigung von künstlichen Gebissen nach einem der Ansprüche 1 bis 8, worin sowohl die körnige Bleichmittelaktivatorzusammensetzung als auch die körnige Bleichmittelzusammensetzung eine Schüttdichte von weniger als 1,0 g/cm$^3$ besitzen.

**10.** Zusammensetzung zur Reinigung von künstlichen Gebissen nach einem der Ansprüche 1 bis 9, welche 1% bis 25% eines polymeren Polycarboxylates als Komplexbildner umfaßt.

**11.** Zusammensetzung zur Reinigung von künstlichen Gebissen nach einem der Ansprüche 1 bis 10, welche Farbteilchen in der Form eines wasserdispergierbaren teilchenförmigen Trägers umfaßt, welcher eine Schüttdichte von mehr als 1,04 g/cm$^3$ aufweist und eine Oberflächenbeschichtung aus Farbstoff oder Pigment besitzt.

**12.** Zusammensetzung zur Reinigung von künstlichen Gebissen nach Anspruch 11, worin der teilchenförmige Träger Kristalle aus Sulfamidsäure umfaßt.

**Revendications**

**1.** Composition granulaire de nettoyage de prothèses dentaires, comprenant un agent de blanchiment de type persel minéral, un précurseur d'agent de blanchiment peracide organique et un générateur d'effervescence, dans laquelle la composition comprend (tous les pourcentages étant rapportés au poids total de la composition),

(a) de 2% à 50% d'une composition granulaire d'activateur de blanchiment ayant un pH en dispersion aqueuse à 1% dans le domaine de 2 à 6,5 et comprenant de 0,1% à 10% d'un précurseur d'agent de blanchiment peracide organique, et

b) de 30% à 98% d'une composition granulaire d'agent de blanchiment ayant un pH dans le domaine de 7,5 à 10,5 et comprenant de 5% à 70% d'agent de blanchiment persel minéral.

2.  Composition de nettoyage de prothèses dentaires selon la revendication 1, dans laquelle la composition granulaire d'activateur de blanchiment comprend un générateur d'effervescence efficace dans des conditions de pH acide qui comprend les deux constituants d'un couple effervescent carbonate ou bicarbonate/acide, pour que la composition granulaire d'agent de blanchiment se dissolve ou se disperse dans l'eau plus lentement ou plus tard que la composition granulaire d'activateur de blanchiment afin de conférer, lors de l'addition à de l'eau de la composition de nettoyage de prothèses dentaires, un pH initial dans le domaine acide.

3.  Composition de nettoyage de prothèses dentaires selon la revendication 2, dans laquelle le pH initial est de 2 à 6,5, de préférence de 3 à 5, et est maintenu dans le domaine acide pendant une durée de 5 secondes à environ 3 heures, de préférence de 10 secondes à 60 minutes, mieux encore de 15 secondes à 30 minutes.

4.  Composition de nettoyage de prothèses dentaires selon l'une quelconque des revendications 1 à 3, dans laquelle la composition granulaire d'agent de blanchiment est présente en excès suffisant par rapport à la composition d'activateur de blanchiment pour déplacer, à la fin de l'effervescence, le pH du milieu aqueux dans le domaine alcalin, de préférence à un pH de 7,5 à 9.

5.  Composition de nettoyage de prothèses dentaires selon l'une quelconque des revendications 1 à 4, dans laquelle la composition granulaire d'agent de blanchiment comprend un générateur d'effervescence efficace dans des conditions de pH alcalin.

6.  Composition de nettoyage de prothèses dentaires selon l'une quelconque des revendications 1 à 5, dans laquelle l'agent de blanchiment persel minéral comprend un ou plusieurs agents de blanchiment choisis parmi les persulfates de métaux alcalins, les perborates de métaux alcalins, et leurs mélanges.

7.  Composition de nettoyage de prothèses dentaires selon la revendication 5 ou 6, dans laquelle le générateur d'effervescence efficace dans des conditions de pH alcalin comprend un perborate de métal alcalin.

8.  Composition de nettoyage de prothèses dentaires selon l'une quelconque des revendications 1 à 7, dans laquelle le précurseur d'agent de blanchiment peracide organique est choisi parmi les polyalkyldiamines acylées, en particulier la tétraacétyléthylènediamine, et les esters carboxyliques de formule générale AcL, dans laquelle Ac est le groupement acyle d'un acide carboxylique organique comprenant un groupement alkyle ou alcényle en $C_6$-$C_{20}$ linéaire ou ramifié, eventuellement substitué, ou un groupement aryle substitué par un ou des groupes alkyle en $C_6$-$C_{20}$, et L est un groupe partant, dont l'acide conjugué possède un pKa dans le domaine de 4 à 13.

9.  Composition de nettoyage de prothèses dentaires selon l'une quelconque des revendications 1 à 8, dans laquelle la composition granulaire d'activateur d'agent de blanchiment et la composition granulaire d'agent de blanchiment ont toutes deux une masse volumique apparente inférieure à 1,0 g/cm$^3$.

10. Composition de nettoyage de prothèses dentaires selon l'une quelconque des revendications 1 à 9, comprenant de 1% à 25% d'un agent séquestrant polycarboxylate polymère.

11. Composition de nettoyage de prothèses dentaires selon. l'une quelconque des revendications 1 à 10, comprenant des grains sous forme de véhicule particulaire dispersable dans l'eau ayant une masse volumique apparente supérieure à 1,04 g/cm$^3$ et portant un enrobage superficiel de colorant ou de pigment.

**12.** Composition de nettoyage de prothèses dentaires selon la revendication 11, dans laquelle le véhicule particulaire comprend des cristaux d'acide sulfamique.